# EUROPÄISCHE PATENTSCHRIFT

(11) **EP 0 570 980 B1**
(45) Veröffentlichungstag und Bekanntmachung des Hinweises auf die Patenterteilung: **30.07.1997**
(21) Anmeldenummer: 93108291.1
(22) Anmeldetag: 21.05.1993
(51) Int. Cl.: A61F 13/15

(54) **Wegwerfwindeln**
Disposable diapers
Couches-culottes à jeter

(30) Priorität: 22.05.1992 JP 130935/92
(43) Veröffentlichungstag der Anmeldung: 24.11.1993
(73) Patentinhaber: UNI-CHARM CORPORATION, Kawanoe-shi Ehime-ken (JP)
(72) Erfinder: Igaue, Takamitsu, Kawanoe-shi, Ehime-ken (JP); Inoue, Kohji, Kanonji-shi, Kagawa-ken (JP); Kido, Tsutomu, Kawanoe-shi, Ehime-ken (JP)
(74) Vertreter: Sperling, Rüdiger, Dipl.-Ing.

(56) Entgegenhaltungen:
- WO-A-88/07337
- GB-A- 2 244 422
- US-A- 4 743 239
- US-A- 5 074 854

## Beschreibung

Diese Erfindung betrifft allgemein eine Wegwerfwindel und insbesondere eine derartige Windel, die zur wahlweisen Verwendung entweder als sogenanntes Windelhöschen mit einem Vorder- und Hinterteil, die miteinander bereits in einem Schritt der Herstellung verbunden wurden und so einen geschlossenen Ring um die Hüfte bilden, oder als sogenannte Windel des offenen Typs mit einem Vorder- und Hinterteil, die mittels Befestigungsklappen erst dann miteinander zu verbinden sind, wenn die Windel tatsächlich einem Träger angelegt wird, eingerichtet ist.

Die japanische Gebrauchsmusteranmeldung Amtsblatt-Nr. 1992-5826 zeigt eine Wegwerfwindel auf, die einen Vorder- und Hinterteil umfaßt, die vorab entlang einem der im Hüftbereich einander gegenüberliegenden Seitenabschnitte miteinander verbunden wurden, aber durch eine bandähnliche Befestigung lösbar entlang dem anderen Seitenabschnitt miteinander verbunden werden können. Andererseits zeigt die japanische Patentanmeldung Amtsblatt-Nr. 1992-89050 eine Wegwerfwindel auf, die einen Vorder- und Hinterteil umfaßt, die zum lösbaren Verbinden mittels eines Klebebandes entlang wenigstens einem Seitenabschnitt des Hüftbereiches verbindbar eingerichtet sind.

Die in der vorstehend genannten japanischen Gebrauchsmusteranmeldung Amtsblatt-Nr. 1992-5826 aufgezeigte Windel ist unabhängig davon, ob der Träger sich in stehender oder liegender Stellung befindet, beim Anlegen an den Träger unpraktisch, da einer der seitlichen Seitenabschnitte permanent geschlossen ist. Die in der vorstehend genannten japanischen Patentanmeldung Amtsblatt-Nr. 1992-89050 aufgezeigte Windel ist insofern nachteilhaft, als daß beim Erweitern der Hüftöffnung, um eine derartige Windel einem Träger anzulegen, wie es normalerweise bei normalen Höschen mit in Ringform geschlossener Hüftlinie getan wird, einer oder beide der seitlich gegenüberliegend angeordneten Seitenabschnitte des Hüftbereiches, die vorab geschlossen wurden, wie das bei normalen Höschen der Fall ist, unbeabsichtigt geöffnet werden können und der erwartete Effekt durch das Schließen der Hüftlinie im vorhinein kann auf die Hälfte vermindert werden, da eine derartige Schließung nur durch Klebeband erfolgt (im Verlauf der Herstellung).

Aus der GB-A-2 244 422 ist eine Wegwerfwindel bekannt, bei der das Vorderteil und Hinterteil im Hüftbereich über Bereiche miteinander verbunden ist, und das Vorderteil und Hinterteil entlang diesen Bereichen bei Bedarf wieder getrennt werden können und über einen ebenfalls im Hüftbereich angeordneten klebebandartigen Verschluß wieder verbunden werden können. Jedoch sind bei dieser Wegwerfwindel die Verbindungs- und gleichzeitig Trennbereiche zwischen Vorder- und Hinterteil nicht über die gesamte Länge zwischen Bein- und Hüftöffnung angeordnet und die Befestigungsklappen zum erneuten Verbinden von Vorder- und Hinterteil erstrecken sich ebenfalls nicht über die gesamte Länge zwischen Bein- und Hüftöffnung.

Ausgehend von dem nächstliegenden Stand der Technik, wie er in der GB-A-2 244 422 beschrieben ist, liegt der Erfindung die Aufgabe zugrunde, eine verbesserte Wegwerfwindel aufzuzeigen, die einen Vorderteil und einen Hinterteil aufweist, die im Verlauf der Herstellung entlang den einander seitlich gegenüberliegenden Seitenabschnitten des Hüftbereichs im voraus miteinander verbunden werden, bei Bedarf jedoch voneinander getrennt und wiederum miteinander verbunden werden können, so daß auf diese Weise die wiederum verbundenen, einander gegenüberliegenden Seitenabschnitte nicht unbeabsichtigt geöffnet werden können, auch wenn die Hüftöffnung erweitert wird, um die Windel einem Träger anzulegen und auch nach dem Wiederverbinden der Seitenabschnitte ein unveränderter Sitz der Windel gewährleistet.

Diese Aufgabe wird erfindungsgemäß dadurch gelöst, daß sich die Verbindungslinien von der Beinöffnung bis zur Hüftöffnung erstrecken, jede der Verbindungslinien einen Widerstand von 1000 g/25,4 mm (Zoll) oder mehr gegen Trennung aufweist, die zwischen dem Vorder- und Hinterteil auftritt, und über die gesamte Länge der Verbindungslinien Trennlinien in der Nachbarschaft dieser vorgesehen sind, so daß der Vorderteil entlang diesen Trennlinien vom Hinterteil abgerissen werden kann; und die Trennlinien in ihrer gesamten Länge von Befestigungslappen überdeckt sind.

Vorzugsweise sind die Verbindungslinien durch Schweißen gebildet und die Trennlinien durch punktweise Schnitte oder Perforationen.

### KURZE BESCHREIBUNG DER ZEICHNUNGEN

Die Erfindung wird anhand eines Beispiels unter Bezug auf die beiliegenden Figuren detaillierter beschrieben, wobei:
- Fig. 1: eine Vorderansicht einer aufgerichteten Windel zeigt, bei der eine der Befestigungsklappen an einem Vorderteil befestigt ist;
- Fig. 2: eine Rückansicht der vollständig aufgerichteten Windel zeigt;
- Fig. 3: eine Teilschnittdarstellung in vergrößertem Maßstab einer Linie zeigt, entlang der der Vorderteil, der Hinterteil und die Befestigungsklappen in der Nähe ihrer seitlich gegentiberliegenden Seitenränder bzw. Basisenden miteinander verbunden sind;
- Fig.4: eine Draufsicht von oben auf die aufgerichtete Windel zeigt;
- Fig. 5: eine Fig. 4 ähnliche Ansicht zeigt, die jedoch die aufgerichtete Windel zeigt, deren einander seitlich gegenüberliegende Seitenabschnitte des Vorderteiles von denjenigen des Hinterteiles abgetrennt sind;
- Fig. 6: eine Draufsicht auf die Innenseite der entfalteten Windel zeigt;
- Fig. 7: eine vergrößerte Schnittdarstellung entlang einer Linie X-X in Fig. 6 zeigt; und
- Fig. 8: eine perspektivische Darstellung zur Erläuterung eines Meßverfahrens der Trennfestigkeit der Verbindungslinie zeigt, das unter Verwendung einer Materialprobe durchgeführt wird.

### BEVORZUGTE AUSFÜHRUNGSFORM DER ERFINDUNG

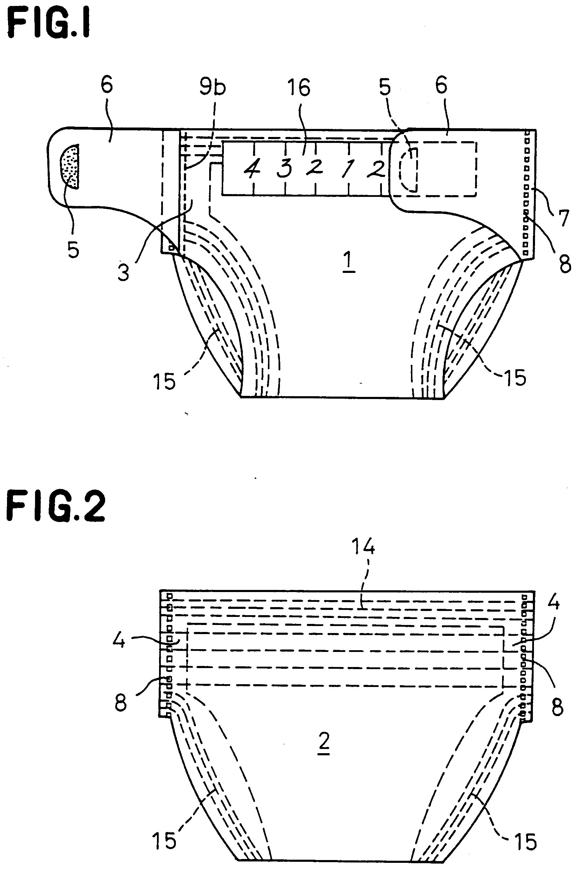

Wie in Fig. 1 bis 5 gezeigt, gehen ein Vorderteil 1 und ein Hinterteil 2 einer Windel in Längsrichtung ineinander über. Vorder- und Hinterteil 1, 2 werden übereinandergefaltet, wobei die einander seitlich gegenüberliegenden Seitenabschnitte 3, 3 des Vorderteiles 1 in der Ebene des Hüftbereiches exakt auf die entsprechenden einander gegenüberliegenden Seitenabschnitte 4, 4 des Hinterteiles 2 gelegt werden, worauf zwei Befestigungsklappen 6, die auf ihren Innenflächen, den vorderen Enden derselben benachbart, Befestigungspunkte 5, die als Befestigungsmittel dienen, tragen, an ihren Basisenden 7 auf die die bereits übereinander gelegten einander seitlich gegenüberliegenden Seitenabschnitte 3, 3 und 4, 4 des Vorder- bzw. Hinterteiles 1, 2 gelegt werden, und diese dergestalt übereinandergelegten Komponenten 1, 2, 6 werden unter der Einwirkung von Wärme oder Ultraschallwellen entlang Linien 8 punktweise miteinander verbunden, die sich parallel zu und unmittelbar den Seitenabschnitten 3, 4 wie auch den Basisenden 7 benachbart erstrecken, so daß die jeweiligen äußeren Ränder dieser Komponenten unverbunden belassen werden. Der Vorderteil 1 ist an den jeweiligen Verbindungslinien 8 benachbarten Stellen 9a mit Trennlinien 9b versehen, die in Form von punktweisen Schnitten oder Löchern (d.h. sogenannten Perforationen) sich parallel zu den jeweiligen Verbindungslinien 8 erstrecken, so daß der Vorderteil 1 entlang diesen Trennlinien 9b vom Hinterteil 2 abgerissen werden kann.

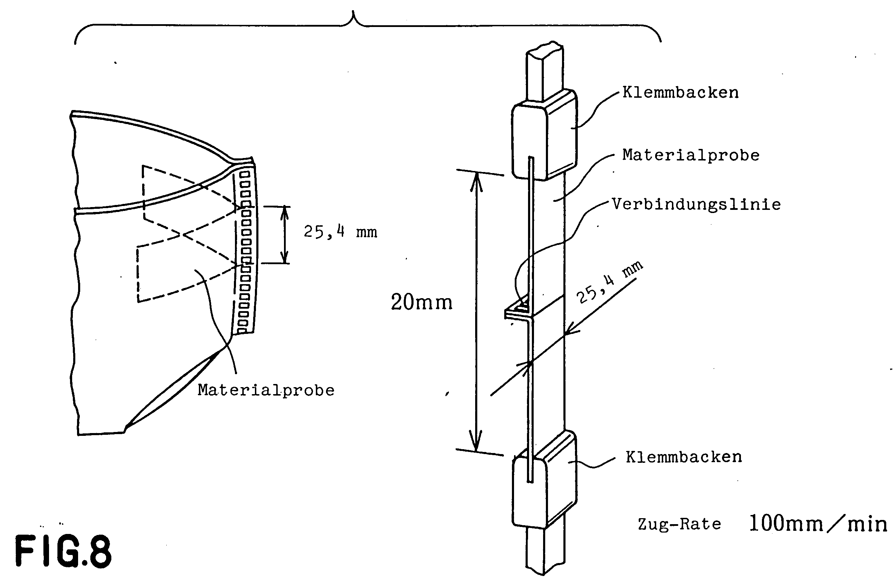

Die Verbindungsfestigkeit (oder der Trennungswiderstand) der jeweiligen Verbindungslinie 8, entlang welcher der Vorder- und Hinterteil 1, 2 miteinander verbunden wurde, sollte vorzugsweise 1000 g/25,4 mm (Zoll) oder mehr betragen, so daß die einander seitlich gegenüberliegenden Seitenabschnitte 3, 3 und 4, 4 der jeweiligen Teile 1, 2, die entlang den Verbindungslinien 8 miteinander verbunden sind, zuverlässig an der Trennung voneinander gehindert sind, auch wenn jede der Verbindungslinien 8 einer Reißkraft unterworfen wird, die möglicherweise zum Trennen der einander seitlich gegenüberliegenden Seitenabschnitte 3, 3 und 4, 4 voneinander neigt, beispielsweise, wenn es erwünscht ist, die durch Verbinden der einander seitlich gegenüberliegenden Seitenabschnitte 3, 3 und 4, 4 in der Ebene des Hüftbereiches des Vorder- und Hinterteiles 1, 2 ausgebildete Hüftöffnung in bereits erwähnter Weise zu erweitern, um die Windel einem Träger anzulegen, oder wenn bei angelegter Windel auf den Vorderteil 1 Zug ausgeübt wird, um den Vorderteil 1 von dem Hinterteil 2 entlang den jeweiligen Trennlinien 9b abzutrennen. Es sei angemerkt, daß der Trennungswiderstand unter Verwendung einer Materialprobe gemessen wird, die ein Teilstück der Verbindungslinie 8 umfaßt und die von der Windel durch Ausschneiden eines in Fig. 8 durch unterbrochene Linien umgebenen Abschnittes entnommen wurde. Wie dargestellt, ist die Materialprobe ein rechteckiger Streifen mit einer Breite von 25,4 mm (1 Zoll), der sich von der Verbindungslinie 8 in entgegengesetzte Richtungen jeweils in Längsrichtung um mehr als 10 mm erstreckt. Die Materialprobe wird an den in Längsrichtung gegenüberliegenden Enden derselben jeweils von Klemmbacken gehalten, so daß sich die Materialprobe anfänglich zwischen den beiden Klemmbacken exakt über einen Abstand von 20 mm erstreckt, wobei die Verbindungslinie 8 in der Mitte liegt. Anschließend wird auf die Materialprobe mit einer Geschwindigkeit von 100 mm/min. in Längsrichtung Zug ausgeübt und dadurch wird ein Wert (g), der im Moment der entlang der Verbindungslinie 8 auftretenden Trennung auftritt, als Trennungswiderstandswert bestimmt.

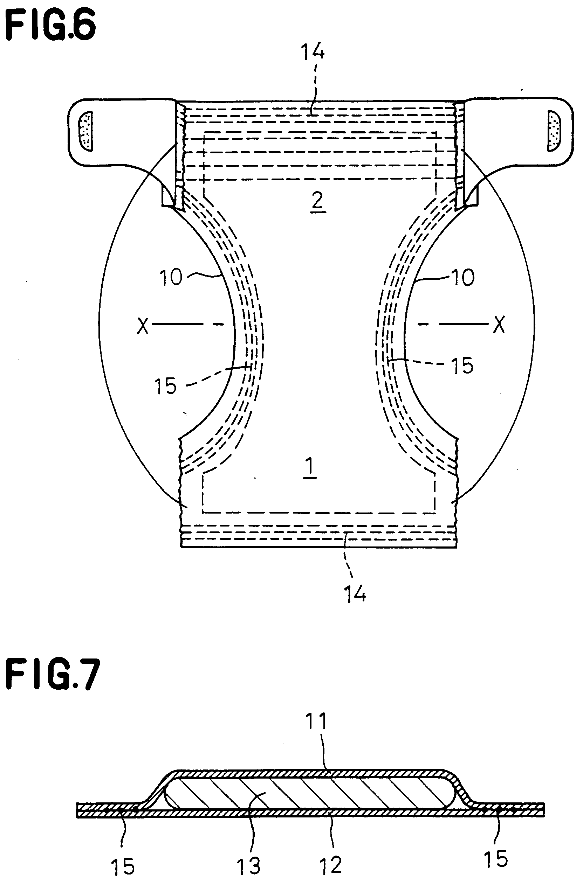

Wie Fig. 1, 2 und 6 zeigen, wird jeder Windelzuschnitt, der den in Längsrichtung ineinander übergehenden Vorder- bzw. Hinterteil 1, 2 umfaßt, anschließend mit einander seitlich gegenüberliegenden konkaven Rändern 10 versehen, die sich zwischen den jeweiligen Hüftbereichen des Vorder- bzw. Hinterteiles 1, 2 erstrecken, um so die jeweiligen Beinöffnungen zu bilden. Dieser Zuschnitt setzt sich, wie in Fig. 7 am besten zu erkennen ist, aus einer flüssigkeitsdurchlässigen Decklage 11, einer flüssigkeitsundurchlässigen Außenlage 12 und einer zwischen die Decklage 11 und die Außenlage 12 gelegten, stundenglasförmigen flüssigkeitsabsorbierenden Platte 13 zusammen. Weiter ist der Zuschnitt entlang den jeweiligen Hüftbereichen des Vorderteiles und Hinterteiles 1, 2 sowie entlang den vorstehenden beschriebenen konkaven Rändern 10, die zur Ausbildung der jeweiligen Beinöffnungen bestimmt sind, mit mehreren parallel zueinander verlaufenden, fadenartigen elastischen Elementen 14, 15 jeweils versehen, die in in Längsrichtung gedehntem Zustand zwischen der Decklage und der Außenlage 11, 12 unter Verwendung von Heißschmelzkleber angebracht sind. Die dem Hüftbereich des Hinterteiles 2 zugehörigen elastischen Elemente 14 sind so angeordnet, daß sie im wesentlichen in diesem gesamten Bereich parallel zueinander mit zunehmenden Zwischenräumen vom Außenrand zum Inneren des Hüftbereiches hin angeordnet sind, so daß die Gesamtelastizität dieser elastischen Elemente 14 höher ist als die der elastischen Elemente 14, die dem Hüftbereich des Vorderteiles 1 zugeordnet sind. Es versteht sich, daß, wenngleich nicht dargestellt, der Hüftbereich des Hinterteiles 2 teilweise aus Stücken elastischen Stoffes hergestellt sein kann, ohne daß sowohl der Vorder- als auch der Hinterteil 1, 2 mit den jeweiligen elastischen Elementen 14 wie in der dargestellten Ausführungsform versehen sind. Beispielsweise können derartige Stücke von elastischem Stoff mittels Schweißung oder ähnlichem an den einander seitlich gegenüberliegenden Seitenrändern des Hüftbereiches des Hinterteiles 2 mit diesem verbunden sein. Obgleich nicht dargestellt, ist es auch möglich, die Befestigungsklappen 6 zur Gänze oder teilweise aus elastischem Material zu bilden, ohne den Umfang der Erfindung zu verlassen.

Der Hüftbereich des Vorderteiles 1 ist mit einem mit einer Skala versehenen Streifen 16 versehen, der auf die Außenfläche desselben geklebt ist, um diese Oberfläche zu schützen, die andernfalls direkt und wiederholt mit den Befestigungspunkten 5 der jeweiligen Befestigungsklappen 6 in Verbindung gebracht würde. Der mit einer Skala versehene schmale Streifen 16 wirkt auch als Anzeigeeinrichtung zur bequemen Anzeige der Stellen, an denen die jeweiligen Befestigungspunkte 5 auf die Außenfläche aufgelegt werden. Die Befestigungspunkte 5 können durch Auftragen von Klebstoff gebildet werden, der herkömmlicherweise für einen derartigen Zweck verwendet wird, oder können ein Stück Band sein, das mit einer Vielzahl von Häkchen versehen ist, wie z.B. Velcro (Warenzeichen) oder Magic Tape (Warenzeichen), die beide dem Fachmann bekannt sind. Um ein derartiges Bandstück als Befestigungspunkt 5 zu verwenden, muß der mit einer Skala versehene schmale Streifen 16 eine Faserfloroberfläche aufweisen, mit der die Häkchen des Befestigungspunktes 5 wirksam in Eingriff bringbar sind.

Die Befestigungsklappe 6 kann aus Vliesstoff oder einer laminierten Lage, die aus derartigem Vliesstoff und Kunststoffolie oder qualitativ hochwertigem Papier besteht, gebildet sein. Die Decklage 11 kann aus Vliesstoff oder poröser Kunststoffolie bestehen. Die Außenlage 12 kann aus Kunststoffolie oder einer laminierten Lage, bestehend aus derartiger Kunststoffolie und Vliesstoff hergestellt sein. Der mit einer Skala versehene schmale Streifen 16 kann aus Kunststoffolie oder Vliestoff oder Filz in Abhängigkeit von der gewünschten Natur des Befestigungspunktes 5 hergestellt sein.

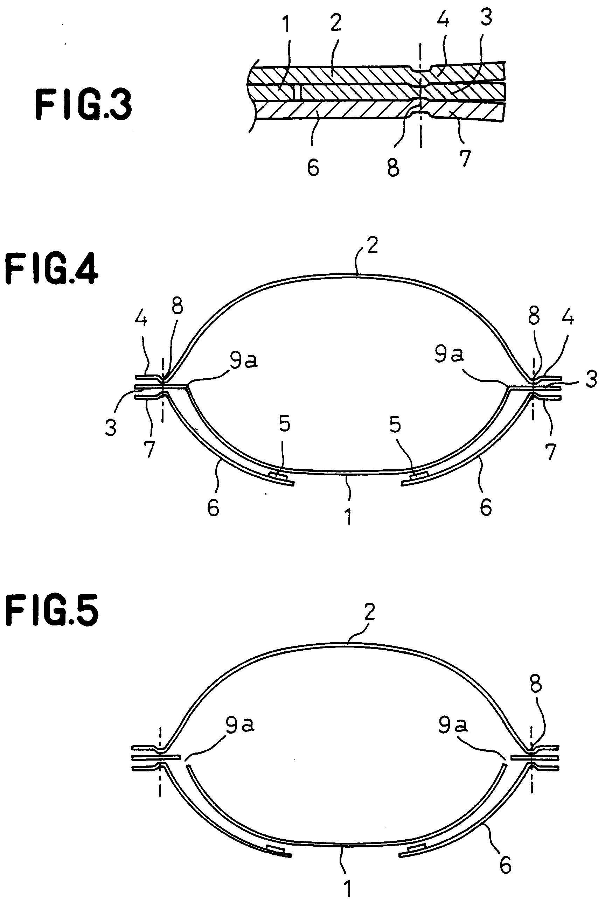

Die gemäß der Lehre dieser Erfindung wie vorstehend beschrieben aufgebaute Windel kann einem Träger als Windel des Höschentyps, d.h. in ihrer ursprünglichen Konfiguration, angelegt werden, beispielsweise, wenn es erwünscht ist, die Windel einem Träger anzulegen, der sich in stehender Stellung befindet. Während die Befestigungsklappen 6 in diesem Fall prinzipiell nicht erforderlich sind, ist es bevorzugt, daß die Befestigungspunkte 5 mit dem schmalen Streifen 16 an den geeigneten Stellen desselben fest in Eingriff stehen, um eine unerwünschte Bewegung der Windel relativ zum Körper des Trägers wahrend ihrer Verwendung zu vermeiden (Fig. 4). Wenn es erwünscht ist, die Windel einem Träger anzulegen, der sich in liegender Stellung befindet, werden die einander seitlich gegenüberliegenden Seitenabschnitte des Vorderteiles 1 von denjenigen des Hinterteiles 2 entlang den Trennlinien 9b abgerissen, indem auf dem Vorderteil 1 oder die Befestigungsklappen 6 Zugkraft ausgeübt wird, um so eine Windel des offenen Typs (Fig. 6) zu erhalten, und nach dem Anlegen an den Träger werden die Befestigungsklappen 6 nach innen auf den schmalen Streifen 16 gefaltet, so daß die jeweiligen Befestigungspunkte 5 mit dem Streifen 16 an gewünschten Stellen fest in Eingriff gebracht werden (Fig. 5).

Die Erfindung ermöglicht es, die Windel einem Träger wahlweise entweder als Höschenwindel oder als offene Windel anzulegen. Die Windel gemäß dieser Erfindung ist insbesondere aus dem Grund vorteilhaft, daß, auch wenn auf die Verbindungslinien, die sich entlang den seitlich gegenüberliegenden Seitenabschnitten erstrecken, eine Zugkraft ausgeübt wird, die normalerweise beim Verwenden der Windel als Höschenwindel ausgeübt wird, entlang den einander seitlich gegenüberliegenden Seitenabschnitten des Vorder- und Hinterteiles keine Abtrennung auftritt, da jede der Verbindungslinien einen Trennungswiderstand aufweist, der wenigstens 1000 g/25,4 mm (Zoll) oder mehr beträgt.

Nach dem Ausscheiden von festen Ausscheidungen auf die Windel kann der Vorderteil vom Hinterteil entlang den Trennlinien abgerissen werden, die entlang den einander seitlich gegenüberliegenden Seitenabschnitten vorgesehen sind, so daß dadurch die Windel leicht dem Träger abgenommen werden kann, ohne daß in unerwünschter Weise die Haut des Trägers mit den festen Ausscheidungen verschmiert wird.

## Patentansprüche

1. Wegwerfwindel, mit in Längsrichtung ineinander übergehenden Vorder- (1) und Hinterteil (2) und zwei von den einander seitlich gegenüberliegenden Seitenabschnitten (3,4) des Hüftbereiches des Vorder- (1) oder Hinterteiles (2) sich nach außen erstreckende Befestigungsklappen (6), wobei jeweils die einander seitlich gegenüberliegenden Seitenabschnitte (3,4) des Vorderteiles (1) exakt auf diejenigen des Hinterteiles (2) gelegt sind;
die Befestigungsklappen (6) an den Innenflächen ihrer jeweiligen Vorderenden Befestigungspunkte (5) tragen und die jeweiligen Basisenden auf die einander seitlich gegenüberliegenden Seitenabschnitte (3) des Hüftbereiches des Vorderteiles (1) aufgelegt sind;
und mit Verbindungslinien (8), entlang welchen die einander seitlich gegenüberliegenden Seitenabschnitte (3,4) des Hüftbereiches des Vorder- (1) und Hinterteiles (2) und die Basisenden der jeweiligen Befestigungsklappen (6) miteinander verbunden sind;
**dadurch gekennzeichnet,** daß
sich die Verbindungslinien (8) von der Beinöffnung bis zur Hüftöffnung erstrecken,
jede der Verbindungslinien (8) einen Widerstand von 1000 g/25,4 mm (Zoll) oder mehr gegen Trennung aufweist, die zwischen dem Vorder-(1) und Hinterteil (2) auftritt,
und entlang deren gesamten Länge Trennlinien (9a,9b) in der Nachbarschaft der Verbindungslinien vorgesehen sind, so daß der Vorderteil (1) entlang diesen Trennlinien (9a,9b) vom Hinterteil (2) abgerissen werden kann
und die Trennlinien (9a,9b) in ihrer gesamten Länge von den Befestigungsklappen (6) überdeckt sind.

2. Wegwerfwindel gemäß Anspruch 1, wobei die Verbindungslinien (8) durch Schweißung gebildet sind und die Trennlinien (9a,9b) durch punktweise Schnitte oder Perforationen gebildet sind.

## Claims

1. A disposable nappy, with front (1) and rear (2) parts running into each other in the longitudinal direction and two fixing flaps (6) extending out from the side sections (3, 4) laterally opposite one another of the hip region of the front (1) or rear (2) part, wherein each of the side sections (3, 4) laterally opposite one another of the front part (1) are disposed exactly on those of the rear part (2);
the fixing flaps (6) carry attachments points (5) on the inner surfaces of their respective leading ends and the respective base ends are applied to the mutually laterally opposite side sections (3) of the hip region of the front part (1);
and with connecting lines (8) along which the mutually laterally opposite side sections (3, 4) of the hip region of the front part (1) and rear part (2) and the base ends of the respective fixing flaps (6) are joined to one another;
characterized in that
the connecting lines (8) extend from the leg opening up to the hip opening,
each of the connecting lines (8) has a resistance to separation of 1000 g/25.4 mm (inch) or more occurring between the front (1) and the rear (2) parts,
and parting lines (9a, 9b) are provided over their whole length in the vicinity of the connecting lines, so that the front part (1) can be torn away from the rear part (2) along these parting lines (9a, 9b),
and the parting lines (9a, 9b) are overlapped by the fixing flaps (6) over their whole length.

2. A disposable nappy according to claim 1, wherein the connecting lines (8) are formed by welding and the parting lines (9a, 9b) are formed by point-wise cuts or perforations.

## Revendications

1. Lange jetable, comprenant une partie avant (1) et une partie arrière (2) qui se rejoignent dans le sens longitudinal, et deux rabats de fixation (6) s'étendant vers l'extérieur depuis les portions latérales mutuellement opposées (3, 4) de la zone de hanche de la partie avant (1) ou arrière (2),
les portions latérales mutuellement opposées (3, 4) de la partie avant (1) étant chaque fois posées exactement sur celles de la partie arrière (2), les rabats de fixation (6) portant des moyens de fixation (5) sur les surfaces internes de leurs extrémités libres respectives, et leurs extrémités de rattachement étant posées sur les portions latérales mutuellement opposées (3) de la zone de hanches de la partie avant (1), lange présentant des lignes de jonction (8) suivant lesquelles on raccorde les portions latérales mutuellement opposées (3, 4) de la zone de hanches des parties avant (1) et arrière (2) et les extrémités de rattachement des rabats de fixation (6), caractérisé en ce que les lignes de jonction (8) s'étendent de l'ouverture de passage des jambes à la ceinture,
chaque ligne de jonction (8) présente une résistance de 1000 g/25,4 mm (pouce) ou plus, à la séparation, qui se produit entre les parties avant (1) et arrière (2),
et on prévoit sur toute la longueur, des lignes de séparation (9a, 9b) au voisinage des lignes de jonction, en sorte que la partie avant (1) puisse être arrachée de la partie arrière (2) en suivant ces lignes de séparation (9a, 9b),
et les lignes de séparation (9a, 9b) sont recouvertes par les rabats de fixation (6) sur toute leur longueur.

2. Lange jetable selon la revendication 1, dans lequel les lignes de jonction (8) sont formées par soudage, et les lignes de séparation (9a, 9b), par des fentes ponctuelles ou des perforations.
